Europäisches Patentamt

European Patent Office

Office européen des brevets

(11) Veröffentlichungsnummer: **0 492 044 B1**

## EUROPÄISCHE PATENTSCHRIFT

(45) Veröffentlichungstag der Patentschrift: **14.06.95**

(21) Anmeldenummer: **91111375.1**

(22) Anmeldetag: **09.07.91**

51 Int. Cl.⁶: **A61B 3/032**, A61B 3/08

54 **Sehtestgerät.**

(30) Priorität: **22.12.90 DE 9017409 U**

(43) Veröffentlichungstag der Anmeldung:
**01.07.92 Patentblatt 92/27**

(45) Bekanntmachung des Hinweises auf die
Patenterteilung:
**14.06.95 Patentblatt 95/24**

(84) Benannte Vertragsstaaten:
**DE ES FR GB IT**

(56) Entgegenhaltungen:
**EP-A- 0 237 687**
**DE-A- 2 543 903**
**US-A- 4 299 455**
**US-A- 4 437 737**

(73) Patentinhaber: **Oculus Optikgeräte GmbH**
**Münchholzhäuserstrasse 29**
**D-35582 Wetzlar (DE)**

(72) Erfinder: **Reiner, Josef, Prof. Dr.**
**Stefan-Lochner-Strasse 14**
**W-5000 Köln 50 (DE)**
Erfinder: **Kirchhübel, Rainer, Dipl.-Ing.**
**Freiherr-vom-Stein-Strasse 18**
**W-6334 Asslar (DE)**

(74) Vertreter: **Missling, Arne, Dipl.-Ing.**
**Patentanwalt**
**Bismarckstrasse 43**
**D-35390 Giessen (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf ein Sehtestgerät gemäß dem Oberbegriff von Anspruch 1.

Ein Gerät zur Prüfung des stereoskopischen Sehens ist in der DE-Patentschrift 24 08 414, ein ähnliches Sehtestgerät auch in dem Zentralblatt für Arbeitsmedizin, Arbeitsschutz, Prophylaxe und Ergonomie, Bd. 30 (1980), Seiten 110 bis 113 beschrieben. Dieses Sehtestgerät weist einen Hohlspiegel auf, in dessen Brennpunkt sich ein Testobjekt befindet. In dem Strahlengang zwischen dem Testobjekt und dem Hohlspiegel ist ein halbdurchlässiger Spiegel angeordnet, auf welchen das Auge des Probanden blickt. Dieses Sehtestgerät soll die Beobachtung von Sehobjekten im freien Raum möglich machen. Durch entsprechende Anwendung des Gerätes kann ein Testobjekt in beliebiger Entfernung von unendlich bis zu einem Nahpunkt, welcher durch die Brennweite des Hohlspiegels gegeben ist, im freien Raum betrachtet werden. Es ergeben sich hierbei relativ natürliche Betrachtungsbedingungen hinsichtlich der Akkomodation und Konvergenz für die Sehweite des Probanden von unendlich bis zu einem Nahabstand von etwa fünf Zentimetern. Dabei wird nur ein einzelnes Testobjekt verwendet, dessen virtuelles Bild in beliebige Entfernungen projiziert werden kann. Die Einstellung auf verschiedene Entfernungen erfolgt dabei durch Verschiebung des Testobjektes relativ zu der Kombination aus teildurchlässigem Spiegel und Hohlspiegel. Der sich hieraus ergebende wesentliche Nachteil besteht darin, daß sowohl der Hohlspiegel als auch der Teilerspiegel unmittelbar in der Nähe des Augenpaares des Probanden angeordnet sind. Hierdurch erfolgt die Betrachtung des Testobjektes unter den gleichen optischen Bedingungen wie bei Verwendung einer Lupe, welche unmittelbar vor dem Auge des Probanden angeordnet ist, wobei das betrachtete Objekt gegenüber der Lupe verschoben wird, um verschiedene optische Entfernungen darzustellen. Die einzelnen Sehzeichen erscheinen dabei, abhängig von der eingestellten Entfernung, unter verschiedenen Winkeln. Dadurch ergeben sich bei der Einstellung der gleiche Sehzeichen unterschiedliche Sehschärfenwerte. Die größer abgebildeten Sehzeichen sind durch den Probanden leichter zu erkennen und täuschen eine bessere Sehschärfe vor. Die Prüfung der Nahsichtigkeit des Probanden wird hierdurch in nicht nachkontrollierbarer Weise verfälscht.

Aus der DE-PS 32 41 958 ist eine Vorrichtung zur Durchführung von Sehtests bekannt, bei welcher zwei unterschiedliche optische Wege vorgesehen sind, welche einmal zur Prüfung der Nahsichtigkeit und einmal zur Prüfung der Fernsichtigkeit verwendbar sind. Bei der Prüfung des Nahsehens ist der optische Weg nicht mit einer Fokussiereinrichtung versehen. Der Nachteil dieser Vorrichtung besteht darin, daß sie relativ groß ausgebildet sein muß und einen erheblichen mechanischen Aufwand erfordert. Weiterhin ist nicht gewährleistet, daß die Blickrichtung des Probanden exakt auf den gewünschten, vorgegebenen Betrachtungswinkel eingestellt ist, so daß die Möglichkeit von Fehluntersuchungen besteht.

Sehtestgeräte der eingangs genannten Art werden zur raschen Prüfung der Sehfunktionen des Augenpaares eines Probanden verwendet. Im einzelnen sind Messungen der binokularen und monokularen Sehschärfe möglich, sowie der Heterophorie, des stereoskopischen Sehens sowie der Farbensichtigkeit. Weiterhin sind derartige Sehtestgeräte vorteilhaft auch zur Überprüfung von Kurzsichtigkeit, Übersichtigkeit oder Alterssichtigkeit verwendbar.

Aus der DE-Offenlegungsschrift 25 43 903 A1 ist es auch bereib bekannt, zwischen dem Testobjekt und dem Hohlspiegel einen teildurchlässigen Teilerspiegel anzuordnen, durch den ein zweiter optischer Weg ausgebildet wird, so daß das Testobjekt nicht mehr durch den Hohlspiegel betrachtet werden muß.

Die bekannten Sehtestgeräte sind meist aus einem einfachen Stereoskop abgeleitet, wobei der Proband durch zwei Sammellinsen blickt, die in einem fixen Abstand zueinander angeordnet sind. Für den Probanden sind Testfiguren oder ähnliches ersichtlich, welche in doppelter Ausführung jeweils in der Brennebene der Linsen angeordnet sind. Auf diese Weise befinden sich die Testbilder der Testfiguren, welche identisch sein müssen, optisch im Unendlichen, so daß das Augenpaar des Probanden die beiden Bilder zu einer Einheit fusionieren kann. Aus der festen Anordnung der beiden Sammellinsen ergeben sich zwar theoretisch die gleichen optischen Betrachtungsverhältnisse, durch die Aberation der Linsen können jedoch Störungen des beidäugigen Sehens auftreten. Ein weiterer Nachteil dieser bekannten Anordnungen besteht darin, daß der Proband in einen dunklen Kasten blickt, in welchem die hellbeleuchteten Testfiguren oder Testobjekte angeordnet sind. Diese werden zwar optisch ins Unendliche abgebildet, der Proband hat jedoch, bedingt durch die Anordnung der Testobjekte in dem Kasten stets den Eindruck der Nähe, wodurch die Wahrnehmung des Probanden verfälscht wird. Auch hierdurch können Störungen des monokularen oder binokularen Sehens auftreten, welche sich durch unscharfe Bilder oder Doppelbilder äußern. Ein weiterer Nachteil besteht darin, daß sich bei dieser Art der Überprüfung der Sehschärfe bei optisch gleich großen Testobjekten unterschiedliche Ergebnisse im Vergleich zu einer ophtalmologischen Prüfung der Sehschärfe in fünf oder sechs Meter Entfernung ergeben.

Zusätzlich zu der psychischen Konvergenz, welche die oben genannten Störungen des Meßergebnisses verursacht, spielt die Instrumenten-Myopie bei derartigen Einblick-Geräten eine Rolle und verursacht beim Betrachten der optisch ins Unendliche abgebildeten Figuren ein unscharfes Sehen. Die Nähe des Testobjektes wird bei derartigen Geräten dadurch simuliert, daß zusätzliche Linsen und Prismen zwischen den fixen Betrachtungslinsen und dem Testobjekt eingebracht werden. Dies regt das Augenpaar zur Akkomodation und Konvergenz an, wodurch sich wiederum neue Fehlerquellen ergeben. Durch die begrenzten Dimensionen des Testgerätes können nur eine bestimmte Anzahl derartiger Zusatzlinsen und Prismen verwendet werden, so daß die Anzahl der einzustellenden Prüfentfernungen stark begrenzt ist.

Der Erfindung liegt die Aufgabe zugrunde, ein Sehtestgerät der eingangs genannten Art weiter zu verbessern, das bei einfachem Aufbau und einfacher, betriebssicherer Anwendbarkeit eine Vielzahl von Sehtest-Möglichkeiten schafft und bei kompaktem Aufbau dem Probanden eine Verwendung bei konstantem Betrachtungswinkel ermöglicht.

Erfindungsgemäß wird die Aufgabe durch die Merkmale im kennzeichnenden Teil von Anspruch 1 gelöst.

Wenn sich das Auge des Patienten im Brennpunkt des Hohlspiegels befindet, ergibt sich ein telezentrischer Strahlengang. Wenn nun Testobjekte gleicher Größe in unterschiedlicher Entfernung vom Hohlspiegel innerhalb der einfachen Brennweite angeordnet werden, so ergeben sich für den Betrachter verschiedene Sehdistanzen mit natürlicher Akkomodations- und Konvergenzeinstellung. Die Testobjekte oder Sehzeichen erscheinen stets unter dem gleichen Winkel und ergeben somit den gleichen Sehschärfenwert, unabhängig von der Einstellentfernung. Die Einstellentfernung ergibt sich von unendlich bis zu einem Nahwert, welcher etwa der Brennweite des Hohlspiegels entspricht. Die hierdurch zu erzielenden optischen Vorteile lassen sich in einem sehr kompakten Gerät verwirklichen, welches beispielsweise auch in Arztpraxen ohne weitere Schwierigkeiten anwendbar ist.

Der zweite Teilerspiegel ist bevorzugterweise gegenüber der Sehachse in der Hauptblickrichtung um etwa 45 Grad geneigt, wobei unter diesem zweiten Teilerspiegel, welcher die freisichtige Beobachtung möglich macht, die optische Anordnung, welche den Hohlspiegel und den ersten Teilerspiegel umfaßt, angeordnet ist. Zusätzlich zu dem sehr kompakten Aufbau, welcher sich aus dieser Anordnung ergibt, ist der freie Blick des Probanden in horizontaler Richtung nicht beeinträchtigt. Ein weiterer Vorteil dieser Ausgestaltung ergibt sich darin, daß die Verschiebung des Testobjektes in horizontaler Richtung erfolgen kann, woraus sich erhebliche mechanische Vorteile ergeben.

Bei entsprechender Dimensionierung des Hohlspiegels kann ein weiterer Teilerspiegel unterhalb der Stellung des bisher vorgesehenen Teilerspiegels vor dem Augenpaar des Probanden angeordnet werden. Durch diese zweite Anordnung des Teilerspiegels ist es möglich, das Bild des nähergerückten Testobjektes in einer Blickrichtung nach unten ohne Veränderung der Kopfhaltung des Probanden zu betrachten. Hierdurch können Probanden, welche eine Sehhilfe, wie etwa eine Brille verwenden, unterschiedliche Bereiche der Sehhilfe prüfen, beispielsweise einen Nahsehbereich bei einem Gleitsichtglas. Es ist hierbei insbesondere günstig, wenn der zweite Teilerspiegel zur Änderung des Einblickwinkels des Probanden schwenkbar ist, so daß mittels nur eines zweiten Teilerspiegels sowohl eine Nahbetrachtungsprüfung als auch eine Weitsichtigkeits-Prüfung möglich ist.

Durch die kontinuierliche Verstellung des Testobjektes von Unendlich bis zu einer sehr kurzen Entfernung ist eine einwandfreie Bestimmung einer Kurzsichtigkeit des Probanden in dem Bereich von Null bis zum reziproken Wert der Nahentfernung möglich. Durch Verwendung von Zusatzgläsern ist auch die Bestimmung einer Übersichtigkeit vorgesehen.

Die verschiedenen Testobjekte können bei der erfindungsgemäßen Anordnung auf einer Zylinderfläche angebracht werden. Dies hat den Vorteil, daß eine Vielzahl von rechteckigen Testfeldern nebeneinander ohne Raumverlust angeordnet werden kann. Befinden sich rechteckige Testfelder hingegen auf einer Kreisplatte, so geht stets Raum verloren, und die Anzahl der Testfelder ist begrenzt. Auch hat die neue Anordnung den Vorteil, daß die erforderliche Lichtquelle innerhalb des zylindrischen Testkranzes plaziert werden kann. Eine Beeinträchtigung der Testfelder durch die Wärmeentwicklung der Lichtquelle kann bei zweckentsprechender Ausführung durchaus vermieden werden.

Um die Außenumfeld-Helligkeit abzudunkeln, ist es zweckmäßig, wenn der zweite Teilerspiegel mit einem Graufilter verbunden ist. Schließlich ist es zur Vermeidung von Bedienfehlern angebracht, daß monokulare Abdeckblenden vorgesehen sind, von denen jeweils eine Abdeckblende in den zweiten optischen Weg selbsttätig eingeschwenkt wird, wenn ein monokulares Testprogramm gewählt wird, und daß ferner ein Rot/Grün-Filter vorgesehen ist, der selbsttätig in den zweiten optischen Weg eingeschwenkt wird, wenn ein Binokulartest nach dem Anaglyphenverfahren gewählt wird. Auf diese Weise ist zunächst sichergestellt, daß bei Monokulartesten eine Abdeckung für das jeweils nicht untersuchte Auge automatisch vorklappt, so daß nur der entsprechend zu prüfende Sehkanal freigegeben wird. Durch das selbsttätige Einschwenken sowohl der Abdeckblenden als auch der Rot/Grün-Filter können keine Verwechslungen mehr vorkommen, etwa, daß bei monokularen Untersuchungen ein Rot/Grün-Filter eingeschwenkt wird. Es kann damit nicht mehr passieren, daß bei Farbtesten ein farbuntauglicher Proband farbtauglich getestet wird.

Ein anderes Trennverfahren mit Streifen, Masken oder dergleichen direkt vor dem Hohlspiegel eingeschwenkt erlaubt die Binokular-Testung (Stereo-, Phorieprüfung etc.) von Testen mit hellem Untergrund und schwarzen Zeichen oder umgekehrt. Auch kann mit dieser Maskenausführung monokular unter binokularen Bedingungen getestet werden, d.h. beide Sehkanäle sind freigegeben, aber zum Beispiel nur in einem Sehkanal werden Optotypen angeboten.

Das erfindungsgemäße Sehtestgerät ist sehr kompakt und klein dimensioniert, so daß es leicht transportiert werden kann. Es empfiehlt sich dabei, einen Hohlspiegel mit möglichst kurzer Brennweite zu verwenden. Um dabei zu vermeiden, daß Abbildungsfehler, beispielsweise Bildfeldwölbungen auftreten, wird bevorzugterweise eine entsprechend dimensionierte Pluslinse direkt vor die Testebene gesetzt, so daß derartige Bildfeldwölbungsfehler ausgeglichen werden können.

Erfindungsgemäß ist die Möglichkeit geschaffen, die Testobjekte auf einer Zylinderfläche anzuordnen. Diese Ausgestaltung weist den besonderen Vorteil auf, daß eine Vielzahl von rechteckigen Testfeldern nebeneinander ohne Raumverlust angeordnet werden können. Wenn sich die rechteckigen Testfelder, wie beim Stand der Technik bekannt, auf einer Kreisplatte befinden, so ist dies stets mit einem Raumverlust verbunden, wobei die Anzahl der Testfelder begrenzt wird. Die erfindungsgemäße Anordnung der Testobjekte auf einer Zylinderfläche weist weiterhin den wesentlichen Vorteil auf, daß innerhalb des zylindrischen Testkranzes eine Lichtquelle angeordnet werden kann, wobei eine Beeinträchtigung der Testfelder durch die Wärmeentwicklung der Lichtquelle nicht erfolgt.

Im folgenden wird die Erfindung anhand von Ausführungsbeispielen in Verbindung mit der Zeichnung beschrieben. Dabei zeigt:

Fig. 1     eine schematische Seitenansicht des erfindungsgemäßen Sehtestgerätes bei Ausgestaltung zur Prüfung der Sehfähigkeit eines Probanden in der Ferne,

Fig. 2     eine Seitenansicht der in Fig. 1 gezeigten Anordnung bei Prüfung der Sehfähigkeit des Probanden in der Nähe und

Fig. 3     eine weitere Ausgestaltungsform des erfindungsgemäßen Sehtestgerätes sowie zwei Schnittansichten entlang der Linie A-A der Fig. 3.

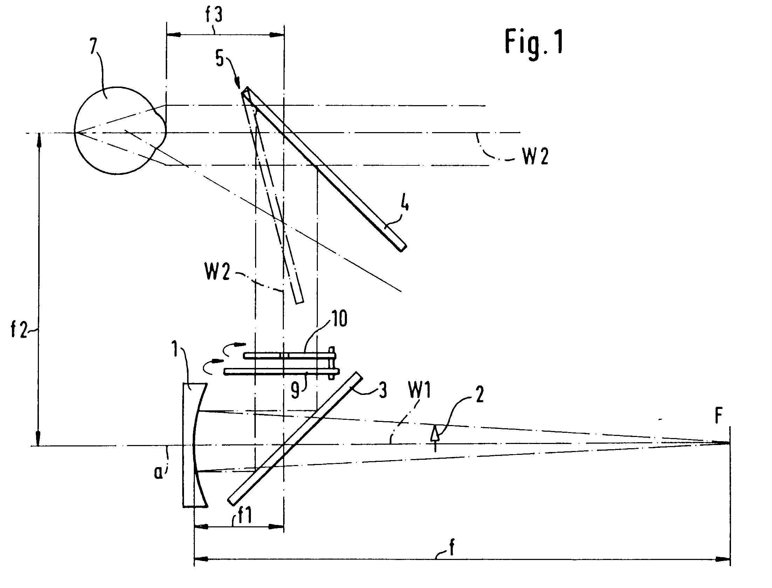

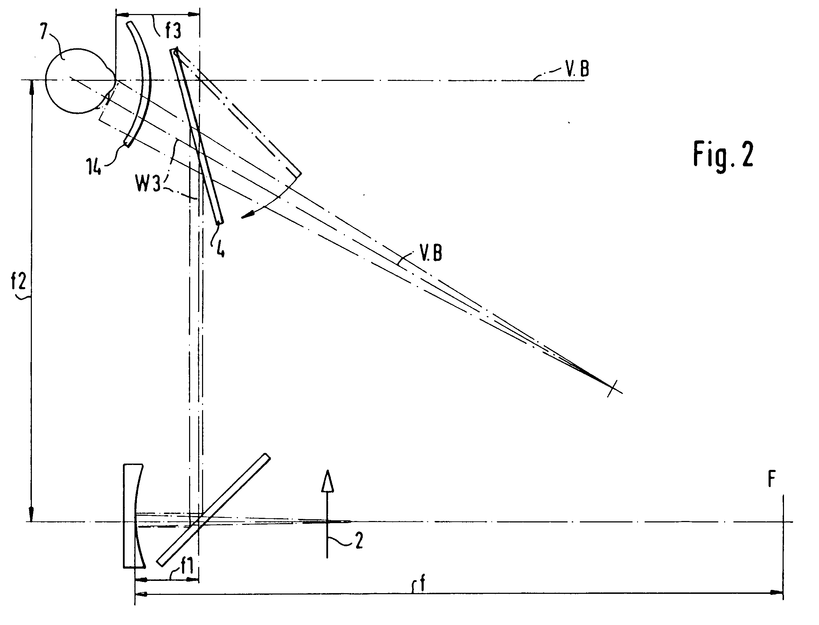

In den Fig. 1 und 2 ist ein Ausführungsbeispiel des erfindungsgemäßen Sehtestgerätes dargestellt, bei welchem ein Hohlspiegel so angeordnet ist, daß dieser einen ersten, waagerechten optischen Weg W1 festlegt. Mit F ist der Brennpunkt des Hohlspiegels 1 angegeben, die Brennweite ist in üblicher Weise mit f dargestellt, wobei die optische Achse des Hohlspiegels mit a bezeichnet ist. Innerhalb der Brennweite f des Hohlspiegels 1 befindet sich ein beleuchtetes Testobjekt 2, welches beispielsweise in Form einer Optotype zu deren reeller Abbildung ausgebildet ist. Direkt vor dem Testobjekt 2 kann eine Zusatzlinse beziehungsweise Linsenkombination angeordnet sein, um Bildfeldwölbungs- und andere Abbildungsfehler auszugleichen (nicht dargestellt).

Das Testobjekt 2 ist innerhalb der Brennweite f längs des optischen Wegs W1 verschiebbar.

Im festen Abstand f1 vom Hohlspiegel 1 ist in der optischen Achse a des Hohlspiegels 1 ein nicht schwenkbarer, halbdurchlässiger, ebener erster Teilerspiegel 3 unter einem Winkel von 45 Grad zur optische Achse a so angeordnet, daß ein Teil der von dem Hohlspiegel 1 zurückgeworfenen Lichtstrahlen senkrecht nach oben abgelenkt wird, wo er auf einen zweiten, ebenfalls halbdurchlässigen ebenen Teilerspiegel 4 auftrifft. Der zweite Teilerspiegel 4 ist um einen Drehpunkt 5 schwenkbar, sowie dies in den Fig. 1 und 2 gezeigt ist. Es ist jeweils die für die Figur zu verwendende Stellung des zweiten Teilerspiegels 4 in durchgezogenen Linien dargestellt, während die andere mögliche Stellung in gestrichelten Linien gezeigt ist.

Der zweite Teilerspiegel 4 kann mit einem Graufilter (nicht dargestellt) versehen sein, um die Außenumfeld-Helligkeit abzudunkeln.

In Fig. 1 ist der zweite Teilerspiegel 4 in einer Stellung dargestellt, in welcher er mit 45 Grad zur optischen Achse a angeordnet ist, so daß die von dem ersten Teilerspiegel 3 ausgehenden Lichtstrahlen in die Horizontale umgeleitet werden und einem Auge 7 eines Probanden zugeführt werden können. Auf diese Weise entsteht ein zweiter optischer Weg W2, welcher zwei zueinander parallele horizontale Teilbereiche sowie einen vertikalen Teilbereich umfaßt. Die Fig. 1, in welcher das Sehtestgerät zur Prüfung der Weitsichtigkeit dargestellt ist, zeigt die einzelnen Abschnitte des optischen Weges W2 und markiert durch die Teil-Brennweiten f1, f2, f3 die Länge des optischen Weges W2. Die Summe der Teil-Brennweiten f1, f2 und f3 ist gleich der Brennweite f des Hohlspiegels 1.

Das Auge des Probanden sieht auf diese Weise bei waagerechter Blickrichtung eine stets scharfe (virtuelle) Abbildung des Testobjektes 2 gegen den Hintergrund des umgebenden Raumes, so daß bei kontinuierlicher Verstellung des Testobjektes 2 von unendlich bis zu einer sehr kurzen Entfernung die einwandfreie Bestimmung einer Kurzsichtigkeit in dem Bereich von Null bis zum reziproken Wert der Nahentfernung möglich ist.

Die Bestimmung einer Übersichtigkeit ist ebenfalls möglich, hierdurch sind in Fig. 1 nicht dargestellte Zusatzgläser zu verwenden.

Die Fig. 1 zeigt weiterhin eine Abdeckblende 9 sowie einen Rot/Grün-Filter 10, welche in den Strahlengang des zweiten optischen Weges W2 wahlweise einbringbar sind. Der Rot/Grün-Filter 10 dient zur Ermittlung der Farbsehfähigkeit des Probanden, während die Abdeckblende 9 so ausgebildet ist, daß jeweils der Strahlengang eines der beiden Augen des Probanden abgedunkelt werden kann, um den stereoskopischen Seheffekt sowie andere Testmöglichkeiten zu überprüfen.

Es versteht sich für den Fachmann, daß die Fig. 1 bis 3 jeweils lediglich den Strahlengang eines Auges des Probanden darstellen, während das Sehtestgerät selbstverständlich zur Testung beider Augen des Probanden ausgebildet ist.

Bei dem in Fig. 2 gezeigten Ausführungsbeispiel ist der zweite Teilerspiegel 4 in seine untere Stellung verschwenkt, so daß sich ein dritter optischer Weg W3 ergibt. Wenn der Proband durch den unteren Bereich einer Zweistärken-Sehhilfe (Brille) blickt, ergibt sich, bedingt durch diesen Nahsichtbereich des Brillenglases 14 eine Neigung der optischen Achse des Auges 7, sowie dieses in Fig. 2 gezeigt ist. Es kann somit ohne weitere Veränderungen des Sehtestgerätes eine Überprüfung der Nahsichtigkeit erfolgen.

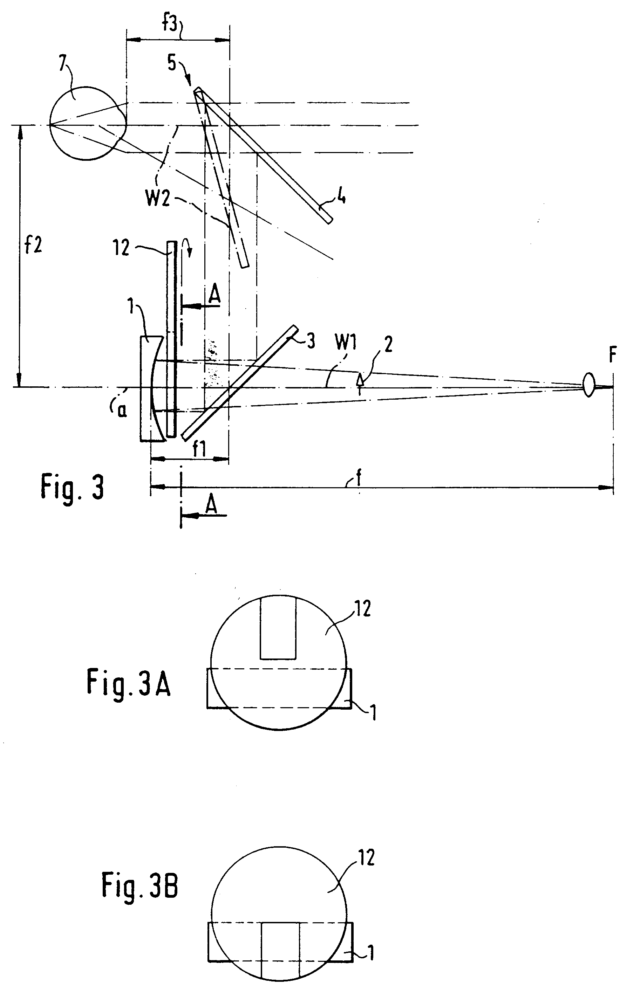

Bei dem in Fig. 3 gezeigten Ausführungsbeispiel ist ergänzend eine Korrekturlinse vorgesehen, um Abbildungsfehler bei Verwendung eines kurzbrennweitigen Hohlspiegels auszugleichen.

Alternativ zu dem Rot/Grün-Filter 10 ist in Fig. 3 ein Maskenrad 12 dargestellt, mit dem je nach Maskenausbildung monokular unter binokularen Bedingungen getestet werden kann und mit dessen Hilfe alle Binokulartest (Stereo-, Phorie-, Fusionstest) angeboten werden können.

Die Erfindung ist nicht auf die gezeigten Ausführungsbeispiele beschränkt, vielmehr ergeben sich für den Fachmann im Rahmen der Erfindung vielfältige Abwandlungs- und Modifikationsmöglichkeiten.

**Patentansprüche**

1. Sehtestgerät mit einem Konkavspiegel (1), einem innerhalb dessen einfacher Brennweite (f) verschiebbar angeordneten Testobjekt (2) und einem zur optischen Achse (a) des Konkavspiegels geneigten, zwischen diesem und dem Testobjekt befindlichen ersten teildurchlässigen Spiegel (3), die so angeordnet sind, daß Licht vom Testobjekt kommend nacheinander durch den ersten teildurchlässigen Spiegel hindurchtritt, am Konkavspiegel reflektiert und durch den ersten teildurchlässigen Spiegel (3) zu einer für das Auge (7) eines Probanden vorgesehenen Position gelenkt wird, welche sich in einem optisch wirksamen Abstand (f1 + f2 + f3) vom Konkavspiegel befindet, der gleich der Brennweite des Konkavspiegels ist, dadurch gekennzeichnet, daß sich zwischen dem ersten teildurchlässigen Spiegel (3) und der für das Auge des Probanden vorgesehenen Position ein zweiter teildurchlässiger Spiegel (4) befindet, welcher das vom ersten teildurchlässigen Spiegel kommende Licht zur für das Auge des Patienten vorgesehenen Position umlenkt.

2. Sehtestgerät nach Anspruch 1, dadurch gekennzeichnet, daß der zweite teildurchlässige Spiegel (4) zur Änderung des Einblickwinkels des Auges (7) des Probanden schwenkbar ist.

3. Sehtestgerät nach Anspruch 1, dadurch gekennzeichnet, daß die teildurchlässigen Spiegel (3, 4) im Strahlengang jeweils so geneigt sind, daß die Einblickrichtung des Auges (7) des Probanden zur optischen Achse des Hohlspiegels (1) parallel ist.

4. Sehtestgerät nach einem der Ansprüche 1 oder 3, dadurch gekennzeichnet, daß die Einblickrichtung der Augen (7) des Probanden und die optische Achse des Konkavspiegels waagerecht verlaufen.

5. Sehtestgerät nach einem der Ansprüche 1 oder 2 dadurch gekennzeichnet, daß der zweite teildurchlässige Spiegel (4) in zumindest zwei Schwenkstellungen so fixierbar ist, daß je nach Stellung des zweiten teildurchlässigen Spiegels der Proband eine in seiner Blickrichtung angeordnete Sehhilfe (14) zumindest in einem Nahsehbereich und einem Fernsehbereich durchblicken kann.

6. Sehtestgerät nach einem der Ansprüche 1 bis 5, dadurch gekennzeichnet, daß der zweite teildurchlässige Spiegel (4) mit einem Graufilter versehen ist, um das durch ihn hindurchtretende Licht der Umgebung abzudunkeln.

7. Sehtestgerät nach einem der Ansprüche 1 bis 6, gekennzeichnet durch eine zwischen dem ersten teildurchlässigen Spiegel und der für das Auge vorgesehenen Position einbringbare Abdeckblende (9).

8. Sehtestgerät nach einem der Ansprüche 1 bis 7, dadurch gekennzeichnet, daß das Sehtestgerät für binokulare Untersuchungen ausgelegt ist.

9. Sehtestgerät nach Anspruch 8, dadurch gekennzeichnet, daß zwischen dem Hohlspiegel (1) und dem Testobjekt (2), bevorzugt in halber Distanz, ein streifen- oder maskenartiges Objekt (12) so angeordnet ist, daß die rechte Hälfte des Testobjektes (2) nur vom rechten Auge (7) und die linke Hälfte des Testobjektes (2) nur vom linken Auge (7) wahrgenommen werden kann.

10. Sehtestgerät nach Anspruch 9, dadurch gekennzeichnet, daß mittels des Objektes (12) nur einzelne Optotypen, Reihen, Zeilen für jeweils eines oder beide Augen freigebbar sind.

11. Sehtestgerät nach einem der Ansprüche 1 bis 10, gekennzeichnet durch einen zwischen dem ersten teildurchlässigen Spiegel und der für das Auge vorgesehenen Position einbringbaren Rot/Grün-Filter (10).

12. Sehtestgerät nach einem der Ansprüche 1 bis 11, dadurch gekennzeichnet, daß vor das Testobjekt (2) eine Plus-Linsenkorrektureinrichtung vorsetzbar ist.

**Claims**

1. A vision testing device having a concave mirror (1), a test object (2) displaceably arranged within the simple focal length (f) of the said concave mirror (1) and a first semi-reflecting mirror (3) inclined at an angle to the optical axis (a) of the concave mirror and located between the latter and the test object, these being arranged in such a manner that light coming from the test object successively passes through the first semi-reflecting mirror, is reflected on the concave mirror and is deflected by the first semi-reflecting mirror (3) into a position provided for the eye (7) of a patient, the said position being at an optically effective distance (f1 + f2 + f3) from the concave mirror equal to the focal length of the concave mirror, characterised in that a second semi-reflecting mirror (4) is located between the first semi-reflecting mirror (3) and the position provided for the eye of the patient and deflects the light coming from the first semi-reflecting mirror into the position provided for the eye of the patient.

2. A vision testing device according to claim 1, characterised in that, in order to change the visual angle of the eye (7) of the patient, the second semi-reflecting mirror (4) is tiltable.

3. A vision testing device according to claim 1, characterised in that the semi-reflecting mirrors (3, 4) are each inclined in the beam path in such a manner that the line of view of the eye (7) of the patient is parallel to the optical axis of the concave mirror (1).

4. A vision testing device according to either one of claims 1 and 3, characterised in that the direction of view of the eyes (7) of the patient and the optical axis of the concave mirror extend horizontally.

5. A vision testing device according to either one of claims 1 and 2, characterised in that the second semi-reflecting mirror (4) is fixable in at least two tilted positions so that the patient, according to the position of the second semi-reflecting mirror, can look through a visual aid (14) arranged in his line of view at least in a close visual range and a distant visual range.

6. A vision testing device according to any one of claims 1 to 5, characterised in that the second semi-reflecting mirror (4) is provided with a grey filter in order to darken the ambient light passing through it.

7. A vision testing device according to any one of claims 1 to 6, characterised by a mask (9) placeable between the first semi-reflecting mirror and the position provided for the eye.

8. A vision testing device according to any one of claims 1 to 7, characterised in that the vision testing device is designed for binocular tests.

9. A vision testing device according to claim 8, characterised in that a strip-type or mask-type object (12) is arranged between the concave mirror (1) and the test object (2), preferably mid-way, in such a manner that the right-hand half of the test object (2) can only be perceived by the right eye (7) and the left-hand half of the test object (2) can only be perceived by the left eye (7).

10. A vision testing device according to claim 9, characterised in that, by means of the object (12), only individual optotypes, rows or lines can be uncovered for either one or both eyes.

**11.** A vision testing device according to any one of claims 1 to 10, characterised by a red/green filter (10) placeable between the first semi-reflecting mirror and the position provided for the eye.

**12.** A vision testing device according to any one of claims 1 to 11, characterised in that a positive lens correction device can be placed in front of the test object (2).

**Revendications**

**1.** Appareil de contrôle de la vue comportant un miroir concave (1), un objet de test (2) monté de manière à pouvoir se déplacer à l'intérieur de la distance focale (f) simple dudit miroir, et un premier miroir partiellement translucide (3) orienté obliquement par rapport à l'axe optique (a) du miroir concave et situé entre ce dernier et l'objet de test, qui sont montés de manière telle que la lumière venant de l'objet de test, successivement, traverse le premier miroir partiellement translucide, se réfléchit sur le miroir concave et est dirigée par l'intermédiaire du premier miroir partiellement translucide (3) vers un emplacement prévu pour l'oeil (7) d'un patient, ledit emplacement étant situé à une distance optiquement efficace (f1 + f2 + f3) du miroir concave qui est égale à la distance focale de ce miroir concave,

caractérisé en ce qu'un deuxième miroir partiellement translucide (4), déviant la lumière arrivant du premier miroir partiellement translucide vers l'emplacement prévu pour l'oeil du patient, est monté entre le premier miroir partiellement translucide (3) et l'emplacement prévu pour l'oeil du patient.

**2.** Appareil de contrôle de la vue selon la revendication 1, caractérisé en ce que le deuxième miroir partiellement translucide (4) est capable de pivoter pour faire varier l'angle d'observation de l'oeil (7) du patient.

**3.** Appareil de contrôle de la vue selon la revendication 1, caractérisé en ce que les miroirs partiellement translucides (3, 4) sont orientés de manière telle sur la trajectoire du faisceau, que la direction d'observation de l'oeil (7) du patient est parallèle à l'axe optique du miroir concave (1).

**4.** Appareil de contrôle de la vue selon l'une quelconque des revendications 1 ou 3, caractérisé en ce que la direction d'observation des yeux (7) du patient et l'axe optique du miroir concave sont horizontaux.

**5.** Appareil de contrôle de la vue selon l'une quelconque des revendications 1 ou 2, caractérisé en ce que le deuxième miroir partiellement translucide (4) peut être fixé dans au moins deux positions de pivotement de manière telle que, en fonction de la position du deuxième miroir partiellement translucide, le patient peut, du moins dans un champ de vision de près et dans un champ de vision de loin, voir à travers un équipement correcteur (14) placé dans sa direction d'observation.

**6.** Appareil de contrôle de la vue selon l'une quelconque des revendications 1 à 5, caractérisé en ce que le deuxième miroir partiellement translucide (4) est pourvu d'un filtre gris, pour assombrir la lumière ambiante le traversant.

**7.** Appareil de contrôle de la vue selon l'une quelconque des revendications 1 à 6, caractérisé par la possibilité de monter un écran de masquage (9) entre le premier miroir partiellement translucide et l'emplacement prévu pour l'oeil du patient.

**8.** Appareil de contrôle de la vue selon l'une quelconque des revendications 1 à 7, caractérisé en ce que l'appareil de contrôle de la vue (7) est conçu pour des analyses binoculaires.

**9.** Appareil de contrôle de la vue selon la revendication 8, caractérisé en ce qu'un objet (12) du type d'une bande ou d'un masque est monté, de préférence à mi-distance entre le miroir concave (1) et l'objet de test (2), de manière telle que la moitié droite de l'objet de test (2) ne peut être perçue que par l'oeil droit (7) et la moitié gauche de l'objet de test (2) ne peut être perçue que par l'oeil gauche (7).

**10.** Appareil de contrôle de la vue selon la revendication 9, caractérisé en ce que l'objet (12) permet de ne valider que certains optotypes et certaines séries ou lignes pour chaque oeil ou pour les deux yeux.

**11.** Appareil de contrôle de la vue selon l'une quelconque des revendications 1 à 10, caractérisé par la possibilité de monter un filtre vert/rouge (10), entre le premier miroir partiellement translucide et l'emplacement prévu pour l'oeil.

**12.** Appareil de contrôle de la vue selon l'une quelconque des revendications 1 à 11, caractérisé en ce qu'il est possible de monter en amont de l'objet de test (2) un dispositif de correction à lentilles convergentes.

Fig.1

Fig. 2

EP 0 492 044 B1

Fig. 3

Fig. 3A

Fig. 3B